# EUROPEAN PATENT SPECIFICATION

(11) **EP 0 618 982 B1**
(45) Date of publication and mention of the grant of the patent: **13.03.1996**
(21) Application number: 92924848.2
(22) Date of filing: 03.12.1992
(51) Int. Cl.: C12Q 1/68

(54) **PROBE FOR AEROMONAS SALMONICIDA AND THE USE THEREOF IN METHODS OF DETECTING OR DETERMINING THE ORGANISM**
Sonde für Aeromonas Salmonicida und dessen Verwendung in Verfahren für den Nachweiss von Organism.
SONDE POUR AEROMONAS SALMONICIDA ET SON UTILISATION DANS DES PROCEDES DE DETECTION OU DE DETERMINATION DE CET ORGANISME

(30) Priority: 04.12.1991 IE 420591
(43) Date of publication of application: 12.10.1994
(73) Proprietor: FORFAS, Dublin 2 (IE); UNIVERSITY COLLEGE GALWAY, Galway (IE); Gannon, Bernard Francis Xavier, Salthill, Galway (IE); Powell, Richard, Salthill, Galway (IE)
(72) Inventor: GANNON, Bernard, Francis, Xavier, Salthill Galway (IE); POWELL, Richard, Salthill Galway (IE)
(74) Representative: Ryan, Anne Mary
(86) International application number: IE9200026
(87) International publication number: WO9311263

(56) References cited:
- EP-A- 0 395 292
- BIO-TECHNOLOGY (N Y) 8 (3), 1990, pp. 233-236. CODEN: BTCHDA ISSN: 0733-222X; BARRY T ET AL. 'A GENERAL METHOD TO GENERATE DNA PROBES FOR MICROORGANISMS.'
- CAN J FISH AQUAT SCI 46 (5), 198, pp. 877-879. CODEN: CJFSDX ISSN: 0706-652X; HENNIGAN M ET AL. "CHARACTERIZATION OF AEROMONAS - SALMONICIDA STRAINS USING DNA PROBE TECHNOLOGY"
- APPL ENVIRON MICROBIOL 58 (3), 1992, pp. 1039-1042. CODEN: AEMIDF ISSN: 0099-2240; HINEY M ET AL. 'DNA PROBE FOR AEROMONAS SALMONICIDA .'

## Description

### Technical Field

This invention relates to a specific probe and the use thereof in studying the ecology of an organism and the etiology of disease caused by said organism. More particularly, the invention relates to a DNA probe specific for *Aeromonas salmonicida* and to the use of said probe in the ecology of *A. salmonicida* and the etiology of furunculosis.

### Background Art

*A. salmonicida* is the causal agent of furunculosis, a disease which affects many species of fish and is of major importance in salmonid farming. The absence of an efficient selective medium and its poor plating efficiency in mixed cultures (McCarthy, D.H., in Aquatic Microbiology, Society for Applied Bacteriology Symposium Series, F.A. Skinner and J.M. Shewan (ed.), Academic Press, New York, N.Y. (1977) 6, 299) has severely hampered the study of the ecology of this organism and the epidemiology of furunculosis. The assumption that the organism is an obligate pathogen of fish (Popoff, M.Y., in N.R. Krieg and J.G. Holt (ed.), Bergey's Manual of Systematic Bacteriology, The Williams and Wilkins Co., Baltimore, (1984) 1, and Richards, R.H. and R.J. Roberts, in R.J. Roberts (ed.), Fish Pathology, Balliere Tindall, London, (1978) 196) may be an artefact of the limited ability of classical bacteriological techniques to detect the organism in natural environments. Studies based on DNA hybridization (Maclnnes, J.I., *et al.* Can. J. Micro. (1979) 25, 579-586) and restriction fragment length polymorphisms (Hennigan, M., *et al.* Can. J. Fish. Aquat. Sci, (1989) 46, 877-879) have shown that *A. salmonicida* is a highly homogenous taxon in the genus *Aeromonas* which is classified as a member of the family *Vibrionaceae.*

A DNA probe specific to *A. salmonicida* would facilitate studies on the ecology of that organism and the etiology of furunculosis.

### Disclosure of Invention

The invention provides a DNA probe specific for *A. salmonicida* corresponding to a DNA fragment having the sequence indicated in the Sequence Listing (SEQ ID NO:1), a sequence complementary thereto or a portion of either sequence.

The probe according to the invention can be generated by a method comprising the following steps:-
a) preparing a gene bank of *A. salmonicida;*
b) screening said gene bank in parallel with radiolabelled total DNA probes from each of *A. salmonicida* and *A. Hydrophila* by defferential hybridization;
c) selecting a clone which hybridizes with said radiolabelled probe from A. *salmonicida* but which does not hybridize with said radiolabelled probe from *A. hydrophila*;
d) isolating the insert from the clone selected in step c); and
e) confirming the specificity of the insert by using said insert as a probe in a further differential hybridization.

This method enables one to isolate a DNA fragment from an A. *salmonicida* genomic DNA library which is specific for all strains of *A. salmonicida* tested to date. the use of such a DNA fragment as a specific DNA probe facilitates a comprehensive study of the ecology of *A. salmonicida* and the etiology of furunculosis.

The insert DNA of the clone selected in step c) is preferably amplified prior to step d). However, amplification may also be carried out prior to the selection step (step c). Amplification is suitably carried out using the polymerase chain reaction (PCR). However, other nucleic acid amplification methods may also be used.

A DNA probe in accordance with the invention preferably contains greater than ten consecutive nucleotides from the sequence shown in the Sequence Listing (SEQ ID NO: 1) or a sequence complementary thereto.

The invention also provides a method of detecting or determining *A. salmonicida* in a locus, which method comprises screening a sample of material taken from said locus, containing or suspected of containing *A. salmonicida,* with a probe as hereinbefore defined.

Suitably a pair of nucleotide sequences is selected from the sequence shown in the Sequence Listing (SEQ ID NO: 1) for the preparation of primers for use in a nucleic acid amplification reaction, for example PCR.

It is found that sensitivity tests performed using PCR in accordance with the method of the invention enable one to detect the genome equivalent of less than three *A. salmonicida* cells as hereinafter described.

The specificity and sensitivity results hereinafter described will illustrate the usefulness of the DNA probe in accordance with the invention in furthering understanding of the habitat(s) of *A. salmonicida* and the mode of transmission of furunculosis.

The method according to the invention can be used to detect or determine *A. salmonicida* generally in the environment in addition to being useful in clinical investigations.

The method according to the invention enables one to carry out investigations on the ecology of *A. salmonicida* and the etiology of furunculosis in a non-invasive or non-destructive manner.

Invasive/destructive investigation was characteristic of previous studies carried out on the organism and disease caused thereby.

Thus the sample of material for use in accordance with the method of detecting or determining *A. salmonicida* in accordance with the invention may be water, a bacterial culture, fish tissue, salmonid faeces or a sample derived from sediment, influent or effluent from a locus used in the rearing of salmonids. Such sediment may be derived from the bottom of cages used in salmon farming, including marine and fresh water salmon farming.

By fish tissue herein is meant both external and internal tissue. For example, *A. salmonicida* has been detected in external mucous on salmon.

Our investigations would indicate that physical contact of a healthy salmonid with a diseased salmonid is not necessary for the transmission of furunculosis. Thus it is deduced that the organism can be transmitted from fish to fish in the water.

The analysis of sediment under cages used in salmonid rearing in accordance with the method of the invention will enable one to determine how long a locus should remain unoccupied once furunculosis has been identified at said locus and, thereby, assist in eradication of the disease.

### Brief description of the Drawings

FIG. 1 is a photograph of an autoradiogram of differential hybridization of *A. salmonicida* total DNA (A) and *A. hydrophila* total DNA (B) to an *A. salmonicida* DNA library;
FIG. 2 is a photograph of an agarose get of two PCR amplified *A. salmonicida* DNA probes (A) side by side an autoradiogram of hybridization of *A. salmonicida* total DNA (B) and *A. hydrophila* total DNA (C) to the two PCR amplified *A. salmonicida* DNA probes;

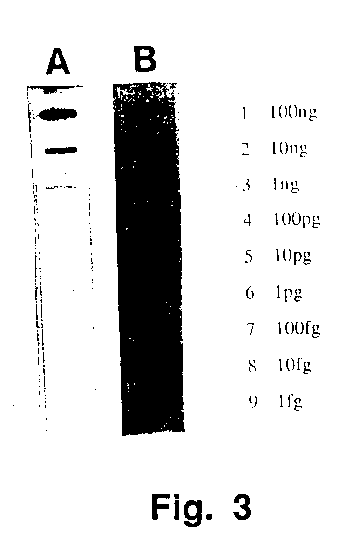

FIG. 3 is a photograph of an autoradiogram of hybridization of a DNA probe for *A. salmonicida* to serial dilutions of *A. salmonicida* total DNA (A) and to the PCR products of said serial dilutions (B).

### Best Mode for carrying out the Invention

The invention will be further illustrated by the following Examples.

### EXAMPLE 1.

### Preparation of total DNA

*Aeromonas* strains were grown on Tryptone Soya Agar/Broth (T.S.A./B.). Total DNA from *Aeromonas* species was prepared as follows. 10 ml. overnight cultures (18-22°C. for *A. salmonicida* strains and 30°C for motile aeromonads) were centrifuged at 5000 rpm for 5 min. and the supernatant discarded. Cells were resuspended in 0.4 ml. solution 1 (50mM Tris. HCl, pH8.0, 50mM EDTA, 25% sucrose). 0.1 ml. lysozyme (10mg/ml in solution 1) was added and the suspension was incubated at 37°C for 15 min.. 0.4 ml. solution 2 (10mM Tris. HCl, pH8.0, 5mM EDTA,1% SDS) was mixed gently with the cell lysate and 0.1 ml. Proteinase K (4mg/ml. in solution 2) was added and incubated at 55°C for 4 h. followed by addition of RNase A (10 mg/ml.) and incubation at 37°C overnight. The DNA was then extracted with phenol/chloroform and precipitated with 2 volumes of isopropanol before resuspending in 0.1 ml. sterile H20. Chemicals were purchased from BDH Ltd. (Poole, England) and the enzymes from Sigma Chemical Company (St. Louis, Mo., U.S.A.).

### EXAMPLE 2

### Preparation of genomic DNA library

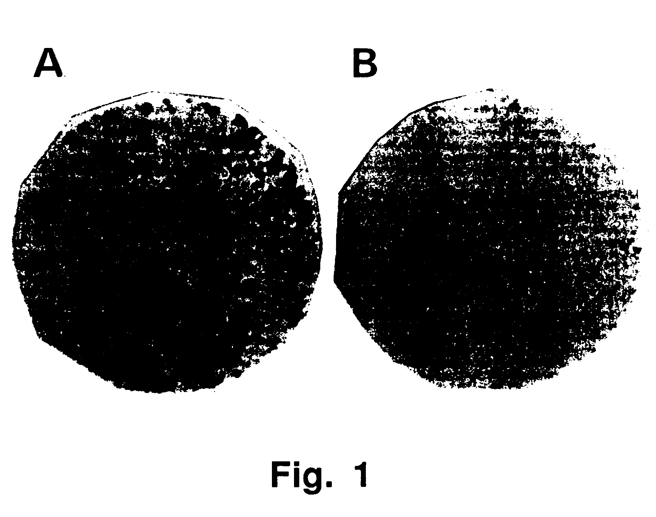

A genomic DNA library was prepared from *EcoRI* digested DNA from *A. salmonicida* strain 7222V (see Table 1) in the vector λgt11(Huynh, T.V. *et al.,* in D.M. Glover (ed.), DNA Cloning, IRL Press, Washington, D.C. (1985)*1*, 49-78, and Maniatis, T., *et al.* Molecular cloning: a laboratory manual, Cold Spring Harbor Laboratory, Cold Spring Harbor, N.Y., (1982)). A library of 10⁶ recombinant phage was generated in *Escherichia coli* Y1090 (Huynh, T.V., *et al.* (1985) *supra).* 10⁴ phage, which is representative of approximately five *A. salmonicida* genome equivalents, were screened *in situ* with [α-³P]dCTP labelled total DNA from *A. salmonicida* strain 7222V and *A. hydrophila* strain AE120 (see Table 1). *A. hydrophila* was chosen for the differential hybridization because a high homology in the respective 16S rRNA sequences had been noted when this approach was examined in relation to obtaining unique DNA sequences for use as specific DNA probes (Barry, T., *et al.* Biotechnology (1990) 8, 233-236). This analysis was also supported by evidence from DNA homology studies which revealed a close genetic relationship between both *Aeromonas* species (Belland, R.J., *et al.* J. Gen. Micro. (1988) *134*, 307-315, Maclnnes, J.I., *et al.* (1979) *supra,* and McCarthy, D.H., Ph.D. thesis, (1978), Council of National Academic Awards, U.K.). Successful differential hybridization was achieved only when the total DNA was reduced to a low molecular weight (less than 2kb) using a four base pair recognition restriction enzyme (e.g. Sau3A or HaeIII) and 1 µg of radiolabelled probe was applied per 132mm diameter nitrocellulose filter. The results showed that under stringent hybridization conditions (5X SSC, 65°C. for 8 h.) and washes (0.2X SSC, 65°C for 30 min.), 50% of the plaques hybridized strongly with the *A. salmonicida* 7222V probe whereas the intensity of the hybridization signals with the *A. hydrophila* AE120 probe were less easy to discriminate (as shown in Fig. 1, A and B). In Fig. 1 "A" represent an *A. salmonicida* 7222V library probed with *A. salmonicida* 7222V radiolabelled total DNA and "B" represents an *A.* *salmonicida* 7222V library probed with *A. hydrophila* AE120 radiolabelled total DNA. The findings presumably are a consequence of sequence divergence, even amongst conserved genes of the related Aeromonads.

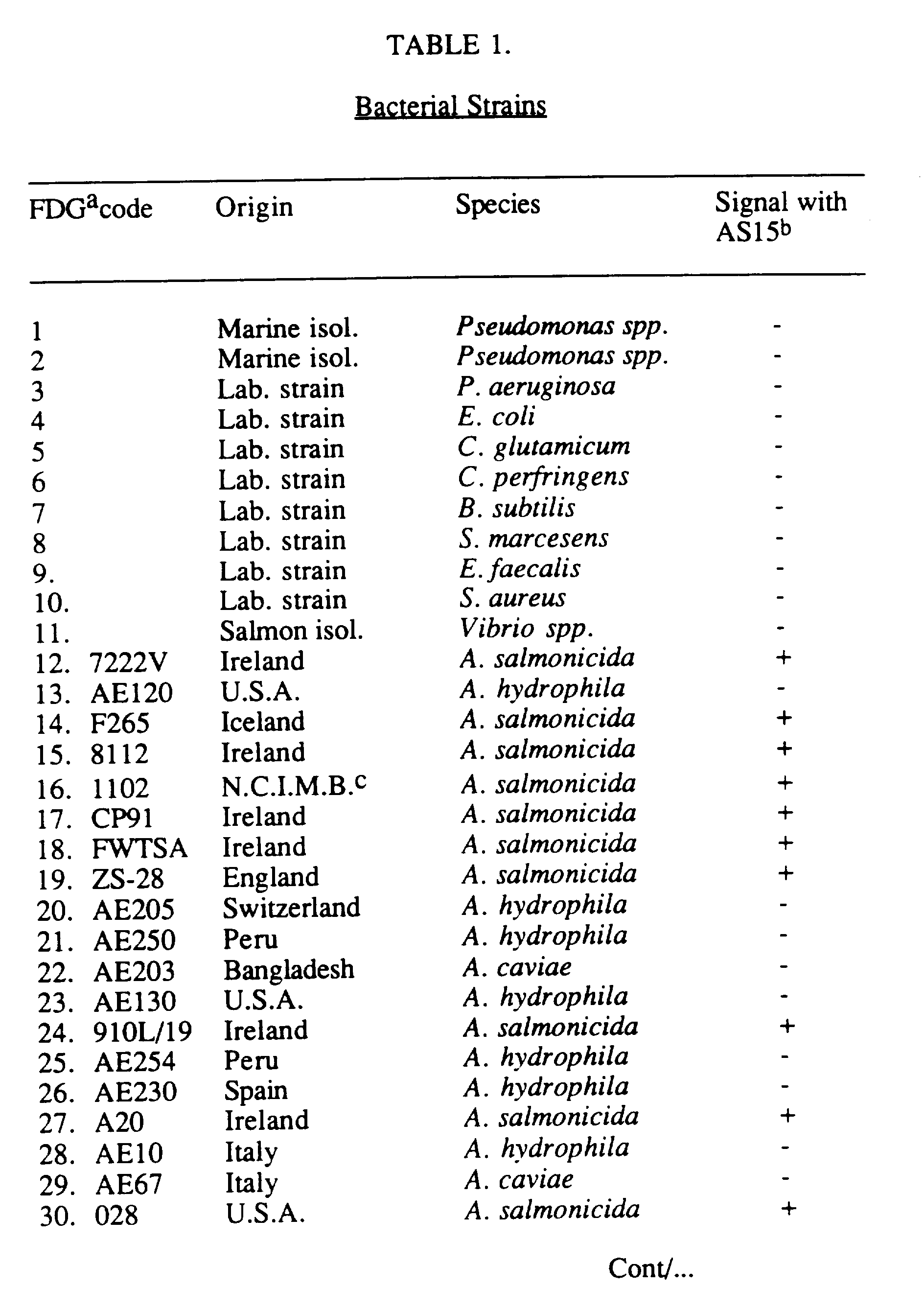

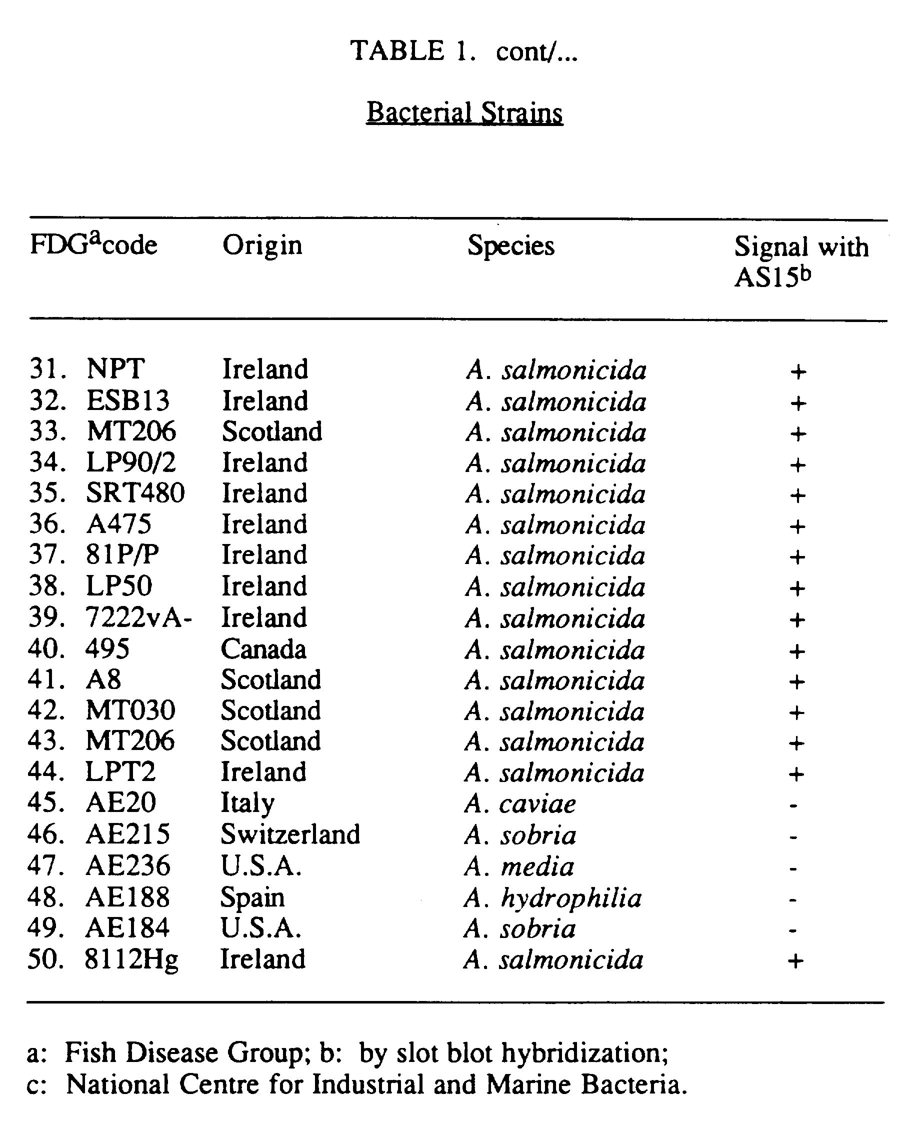

### EXAMPLE 3

### Selection of clones

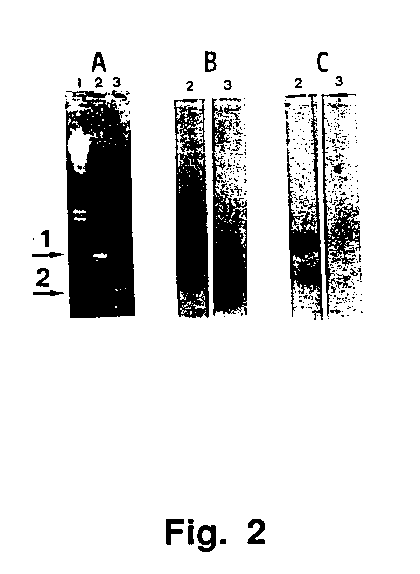

Two clones from Example 2 were chosen for further analysis; AS15 which showed a strong hybridization signal with the *A. salmonicida* 7222V probe and no signal with the *A. hydrophila* AE120 probe and AS7 which hybridized with both probes. The inserts from AS15 and AS7 were amplified using PCR and primers complementary to sequences flanking the EcoRI cloning site of λgt11(Trahey, M., *et al.* Science, (1988) *242*, 1697-1700). The amplification reaction conditions were: 10mM Tris HCl pH 8.3, 1.5mM MgCl₂, 50mM KCl, 200µm dNTPs, 200pmol of each primer (λgt11 forward and reverse sequencing primers; Boehringer Mannheim GmbH, Germany) and 1.5 U Taq polymerase (Promega Corp., Madison USA). Reaction volume was 100µl and cycling conditions were 94°C for 1 min. (denaturation), 50°C for 1 min. (annealing) and 72°C for 1min. (extension); 30 cycles were performed. The results show that clones AS7 and AS15 contain inserts of 0.7kb and 0.5kb respectively as shown in Fig. 2. In Fig. 2 "A" represents PCR amplified AS7 (lane 2) and AS15 (lane 3) inserts separated on a 1% agarose get along with λHindIII mol. wt. size markers (lane 1). A secondary differential screening of southern blots of both fragments, performed under identical hybridization and washing conditions to the *in situ* screening described above, confirmed that AS7 hybridized with total DNAs from both *A. salmonicida* 7222V and *A. hydrophila* AE120 whereas AS 15 was positive only with *A. salmonicida* 7222V as shown in Fig. 2. In Fig. 2 "B" represents a Southem blot of the get probed with *A. salmonicida* 7222V radiolabelled total DNA and "C" represents a Southern blot of the get probed with *A. hydrophila* AE120 radiolabelled total DNA. Also in Fig. 2 arrow 1 shows the AS7 amplified DNA fragment and arrow 2 shows the AS15 amplified fragment.

### EXAMPLE 4

### Analysis of the specificity of the AS15 fragment

To further analyse its specificity, the AS15 fragment of Example 3 was used as a [α-³P]dCTP radiolabelled probe in a slot-blot hybridization using genomic DNAs prepared from typed and clinical isolates of *A. salmonicida,* related Aeromonads and several other bacterial genera (Table 1). These included strains of *Escherichia coli, Pseudomonas aeroginosa, Corynebacterium glutamicum, Clostridium perfringens, Serratia marcesens, Bacillus subtilus, Staphylococus aureus, Enterococcus faecalis* and marine and salmon isolates identified as *Pseudomonas* and *Vibrio* species, respectively. Aeromonad members analysed included different strains of *A. hydrophila, A. caviae, A. sobria and A. media. A. salmonicida* strains included typed strains, clinical disease isolates from Ireland, England, Scotland and the U.S.A. and an *A. salmonicida* sub-species achromogens from Iceland.

Typically, 1µg of total DNA was denatured by addition of a half volume of 1 M NaOH and incubation at 55°C for 5 min. Samples were then neutralised by the addition of an equal volume of 1M HCl prior to application onto Nytran (Nytran is a Trade Mark) membrane (Schleicher & Schuell) using a Schleicher & Schuell slot-blot apparatus. 1µg of radiolabelled AS15 probe was used in a standard hybridization (5X SSC, 5X Denhardts, 0.1% SDS) for 8 h. at 65°C.. The stringency of the final wash was 0.2X SSC for 30 min. at 65°C.. The results showed that the probe hybridized strongly to DNAs from all *A. salmonicida* strains tested, however no signal could be detected with other bacterial genera or with fellow members of the *Aeromonas* family as shown in Table 1.

### EXAMPLE 5

### Analysis of the sensitivity of the AS15 fragment

The results obtained in Example 4 demonstrated that a specific DNA probe for *A. salmonicida* had been isolated. To determine whether this DNA probe might prove a useful tool in the study of the ecology of A. *salmonicida* and the epidemiology of furunculosis the detection limits of the DNA probe were determined. Slot blot analysis of serial dilutions of A. *salmonicida* 7222V DNA revealed that 1µg of radiolabelled AS15 fragment detected 10 ng of total DNA after 8 h. autoradiography as shown in Fig. 4 "A". In Fig. 4 "A" represents direct hybridization against serial dilutions of *A. salmonicida* total DNA. This is equivalent to the DNA content of 2.4 X 10⁶ *A. salmonicida* cells (Belland R.J. *et al. supra*). As this degree of sensitivity is insufficient for an environmental study on *A. salmonicida*, a PCR based detection system was developed. The nucleotide sequence of the AS15 fragment was determined after subcloning into the vector M13 mp18 (Yanisch-Perron, C., *et al*. Gene, (1985) *33*, 103-119) and sequencing using dideoxynucleotides (Sanger, F., *et al.* Proc. Natl. Acad. Sci. USA, (1977) *74*, 5463-5467). Comparison of the DNA sequence with the GenBank (GenBank is a Registered Trade Mark) sequence data library revealed no significant homologies. Comparison of inferred protein sequences from several small open reading frames found in AS15 with the translated products of the GenBank sequence data library also revealed no significant homologies.

Primers for PCR were designed from the AS15 sequence and had the following nucleotide sequences:
Primer 1 : (SEQ ID NO: 2 in the Sequence Listing)
Primer 2: (SEQ ID NO:3 in the Sequence Listing)

These primers sepcifically amplify a 423 bp DNA fragment from DNA prepared from *A. salmonicida* strains and all amplified fragments hybridize with an oligodeoxynucleotide complementary to an internal sequence in AS15 (data not shown). In a comparable sensitivity experiment to that described above, similar serial dilutions of *A. salmonicida* 7222V DND were prepared and amplified by PCR using the designed primers. The amplification reaction conditions were: 10mM Tris HCl pH 8.3,3.5 mM MgCl₂, 50mM KCl, 200 µM dNTPs, 200pmol of each primer and 1.5 U Taq polymerase (Promega Corp., Madison USA). Reaction volume was 100µl and cycling conditions were 94°C for 1 min. (denaturation), 55°C for 1 min. (annealing) and 72°C for 1 min. (extension); 30 cycles were performed. The amplification products were slot blotted as described and probed with 1 µg of radiolabelled AS15 fragment under a similar hybridization and washing regime to that stated earlier. The sensitivity of detection was increased by approximately 10⁶ times, i.e. 10 fg of total DNA gave a positive signal after 4 h. autoradiography as shown in Fig. 4 "B". In Fig. 4 "B" represents hybridization against the PCR products amplified from the individual serial dilutions of *A. salmonicida* total DNA. This is equivalent to the DNA content of 2.4 A. *salmonicida* cells (Belland, R.J. *et al. supra*).

Based on the specificity and sensitivity results presented in Examples 4 and 5 and the current lack of methods to specifically define and cultivate *A. salmonicida*, particularly from environmental samples, the DNA probe in accordance with the invention should now prove a very useful tool in furthering one's understanding of the habitat(s) of *A. salmonicida* and the mode of transmission of furunculosis.

### SEQUENCE LISTING

### (1) ) GENERAL INFORMATION:

(i) APPLICANT:
   (A) NAME: EOLAS (trading as BioResearch Ireland) - The Irish Science and Technology Agency
   (B) STREET: Old Finglas Road, Glasnevin
   (C) CITY: Dublin 9
   (E) COUNTRY: Ireland
   (F) POSTAL CODE (ZIP): None
   (G) TELEPHONE: +353 1 37 01 77
   (H) TELEFAX: +353 1 37 01 76
   (I) TELEX: 32501 EI

   (A) NAME: GANNON, BERNARD FRANCIS XAVIER
   (B) STREET: "Murgan", Pollnarooma, Salthill
   (C) CITY: Galway
   (E) COUNTRY: Ireland
   (F) POSTAL CODE (ZIP): None
   (G) TELEPHONE: +353 91 66559
   (H) TELEFAX: +353 91 66570
   (I) TELEX: 50023 U.C.G. E.I.

   (A) NAME: POWELL, RICHARD
   (B) STREET: Glenard Crescent, Salthill
   (C) CITY: Galway
   (E) COUNTRY: Ireland
   (F) POSTAL CODE (ZIP): None
   (G) TELEPHONE: +353 91 24411 Ext. 2404
   (H) TELEFAX: +353 91 25700
   (I) TELEX: 50023 U.C.G. E.I.

   (A) NAME: UNIVERSITY COLLEGE GALWAY
   (B) STREET: University Road
   (C) CITY: Galway
   (E) COUNTRY: Ireland
   (F) POSTAL CODE (ZIP): None
   (G) TELEPHONE: +353 91 24411
   (H) TELEFAX: +353 91 25700
   (I) TELEX: 50023 U.C.G. E.I.
(ii) TITLE OF INVENTION: Probe for *Aeromonas salmonicida* and the use thereof in methods of detecting or determining the organism
(iii) NUMBER OF SEQUENCES: 3
(iv) COMPUTER READABLE FORM:
   (A) MEDIUM TYPE: Floppy disk
   (B) COMPUTER: IBM PC compatible
   (C) OPERATING SYSTEM: PC-DOS/MS-DOS
   (D) SOFTWARE: PatentIn Release #1.0, Version #1.25 (EPO)
(v) PRIOR APPLICATION DATA
   (A) APPLICATION NUMBER: IE 4205/91
   (B) FILING DATE: DECEMBER 4, 1991

### (2) INFORMATION FOR SEQ ID NO: 1:

(i) SEQUENCE CHARACTERISTICS:
   (A) LENGTH: 455 base pairs
   (B) TYPE: nucleic acid
   (C) STRANDEDNESS: both
   (D) TOPOLOGY: linear
(ii) MOLECULAR TYPE: DNA (genomic)
(iii) HYPOTHETICAL: NO
(iii) ANTI-SENSE: NO
(vi) ORIGINAL SOURCE:
   (A) ORGANISM: *Aeromonas salmonicida*
   (B) INDIVIDUAL ISOLATE: 7222V
   (C) CELL TYPE: unicellular organism
(vii) IMMEDIATE SOURCE:
   (A) LIBRARY: genomic
   (B) CLONE: 7222V/AS15
(xi) SEQUENCE DESCRIPTION: SEQ ID NO: 1:
   TAGAGTTTTG CCGACGCATG GTGCTACAGA CTTTCACTAT    40
   ATAAATACTA TAATTTTTAT TTTGGGTTTT TAAATTAAAA    80
   TAAATTTTAA CTACGAGTTT TAAGGCAAAA CTTTACTGTT   120
   ACACTTTTTC CTTCCGCAAT ACTTATATGG GGGGTAGGTA   160
   TATTTTTGGC TGGATTGGTT GGTTCTACTT TTTTGGTGTT   200
   AGGTTCTCCG TTGATTTCTT CTTAACGTTG AAAACTAACG   240
   TTGGTACCTT TCTCTCAACC GATCGCCACT CAAAAAGTGG   280
   AGTGTACTTT TAATGCGGTC GTATCTAGAT CCATTTTACG   320
   GTAACGAATA GCTCCGTCGG TTGTTATTCG TACACCCCAA   360
   TTTAAACTTA AAACACAGCT TGGATTATCT GAATGGATCA   400
   CTCTATCCTT CTCGGTATAG GTTGCCTTTT CTTGTTAAGT   440
   CTAATGCACC ATTTG                              455

### (2) INFORMATION FOR SEQ ID NO: 2:

(i) SEQUENCE CHARACTERISTICS:
   (A) LENGTH: 20 base pairs
   (B) TYPE: nucleic acid
   (C) STRANDEDNESS: both
   (D) TOPOLOGY: linear
(ii) MOLECULAR TYPE: DNA (genomic)
(iii) HYPOTHETICAL: NO
(iii) ANTI-SENSE: NO
(vi) ORIGINAL SOURCE:
   (A) ORGANISM: *Aeromonas salmonicida*
   (B) INDIVIDUAL ISOLATE: 7222V
   (C) CELL TYPE: unicellular organism
(vii) IMMEDIATE SOURCE:
   (A) LIBRARY: genomic
   (B) CLONE: 7222V/AS15
(xi) SEQUENCE DESCRIPTION: SEQ ID NO: 2:
   CGTTGGATAT GGCTCTTCCT 20

### (2) INFORMATION FOR SEQ ID NO: 3:

(i) SEQUENCE CHARACTERISTICS:
   (A) LENGTH: 20 base pairs
   (B) TYPE: nucleic acid
   (C) STRANDEDNESS: both
   (D) TOPOLOGY: linear
(ii) MOLECULAR TYPE: DNA (genomic)
(iii) HYPOTHETICAL: NO
(iii) ANTI-SENSE: NO
(vi) ORIGINAL SOURCE:
   (A) ORGANISM: *Aeromonas salmonicida*
   (B) INDIVIDUAL ISOLATE: 7222V
   (C) CELL TYPE: unicellular organism
(vii) IMMEDIATE SOURCE:
   (A) LIBRARY: genomic
   (B) CLONE: 7222V/AS15
(xi) SEQUENCE DESCRIPTION: SEQ ID NO: 3:
   CTCAAAACGG CTGCGTACCA 20

## Claims

1. A DNA probe specific for A. *salmonicida* corresponding to a DNA fragment having the sequence indicated in the Sequence Listing (SEQ ID) NO: 1), a sequeiice complementary thereto or a portion of either sequence.

2. A DNA probe according to Claim 1, which contains greater than ten consecutive nucleotides from the sequence indicated in the Sequence Listing (SEQ ID NO: 1) or a sequence complementary thereto.

3. A method of detecting or determining *A. salmonicida* in a locus, which method comprises screening a sample of material taken from said locus, containing or suspected of containing A. *salmonicida,* with a probe according to Claim 1 or 2.

4. A method according to Claim 3, wherein a pair of nucleotide sequences is selected from the sequence as indicated in the Sequence Listing (SEQ ID NO: 1) for the preparation of primers for use in a nucleic acid amplification reaction.

5. A method according to Claim 3 or 4, wherein the sample is water.

6. A method according to Claim 3 or 4, wherein the sample is a bacterial culture.

7. A method according to Claim 3 or 4, wherein the sample is fish tissue.

8. A method according to Claim 3 or 4, wherein the sample is derived from salmonid faeces.

9. A method according to Claim 3 or 4, wherein the sample is derived from sediment from a locus used in the rearing of salmonids.

10. A method according to Claim 3 or 4, wherein the sample is derived from particulate matter in the influent or effluent of a locus used in the rearing of salmonids.

## Patentansprüche

1. DNA-Sonde, spezifisch für A. salmonicida, entsprechend einem DNA-Fragment mit der in der Sequenzliste (SEQ ID Nr. 1) angegebenen Sequenz, einer hierzu komplementären Sequenz oder einem Teil von einer der beiden Sequenzen.

2. DNA-Sonde nach Anspruch 1, die mehr als zehn aufeinanderfolgende Nucleotide aus der in der Sequenzliste (SEQ ID Nr. 1) angegebenen Sequenz enthält, oder aus der hierzu komplementären Sequenz.

3. Verfahren zum Nachweis oder zur Bestimmung von A. salmonicida an einem Locus, wobei das Verfahren das Screenen einer Probe eines Materials aus diesem Locus, der A. salmonicida enthält oder an dem A. salmonicida vermutet wird, mit einer Sonde gemäss Anspruch 1 oder 2 umfasst.

4. Verfahren nach Anspruch 3, worin ein Paar von Nucleotidsequenzen ausgewählt ist aus den Sequenzen wie in der Sequenzliste (SEQ ID Nr. 1) angegeben, zur Herstellung von Primer für die Verwendung in einer Nucleinsäure-Amplifikationsreaktion.

5. Verfahren nach Anspruch 3 oder 4, worin die Probe Wasser ist.

6. Verfahren nach Anspruch 3 oder 4, worin die Probe eine Bakterienkultur ist.

7. Verfahren nach Anspruch 3 oder 4, worin die Probe Fischgewebe ist.

8. Verfahren nach Anspruch 3 oder 4, worin die Probe aus Salmonidkot stammt.

9. Verfahren nach Anspruch 3 oder 4, worin die Probe aus einem Sediment an einem Locus für die Aufzucht von Salmoniden stammt.

10. Verfahren nach Anspruch 3 oder 4, worin die Probe von teilchenförmiger Materie im Einlass oder Auslass eines Locus für die Aufzucht von Salmoniden stammt.

## Revendications

1. Sonde d'ADN spécifique pour A. *salmonicida,* correspondant à un fragment d'ADN ayant la séquence indiquée dans la Liste des Séquences (SEQ ID NO : 1), une séquence complémentaire de celle-ci ou une partie de l'une quelconque de ces séquences.

2. Sonde d'ADN selon la revendication 1, qui contient plus de dix nucléotides consécutifs à partir de la séquence indiquée dans la Liste des Séquences (SEQ ID NO : 1), ou une séquence complémentaire de celle-ci.

3. Procédé de détection ou de détermination de A. *salmonicida* dans un locus, le procédé consistant à cribler un échantillon de matériau prélevé dudit locus, contenant ou susceptible de contenir A. *salmonicida,* avec une sonde selon la revendication ou 2.

4. Procédé selon la revendication 3, dans lequel on choisit une paire de séquences nucléotidiques dans la séquence telle qu'indiquée dans la Liste des Séquences (SEQ ID NO : 1) pour la préparation d'amorces destinées à être utilisées dans une réaction d'amplification d'acide nucléique.

5. Procédé selon la revendication 3 ou 4, dans lequel l'échantillon est de l'eau.

6. Procédé selon la revendication 3 ou 4, dans lequel l'échantillon est une culture bactérienne.

7. Procédé selon la revendication 3 ou 4, dans lequel l'échantillon est du tissu de poisson.

8. Procédé selon la revendication 3 ou 4, dans lequel l'échantillon est issu de selles de salmonidés.

9. Procédé selon la revendication 3 ou 4, dans lequel l'échantillon est est issu de sédiments provenant d'un locus utilisé pour l'élevage de salmonidés.

10. Procédé selon la revendication 3 ou 4, dans lequel l'échantillon est issu d'une substance particulaire dans l'affluent ou l'effluent d'un locus utilisé pour l'élevage de salmonidés.
